# EUROPEAN PATENT SPECIFICATION

(11) **EP 2 666 874 B1**
(45) Date of publication and mention of the grant of the patent: **27.05.2015**
(21) Application number: 13181096.2
(22) Date of filing: 07.07.2008
(51) Int. Cl.: C12Q 1/68, G01N 33/48, C12M 1/34

(54) **Compositions and methods for diagnosing and assessing inflammatory myopathies**
Zusammensetzungen und Verfahren zur Diagnose und Beurteilung entzündlicher Muskelerkrankungen
Compositions et méthodes permettant de diagnostiquer et d'évaluer des myopathies inflammatoires

(30) Priority: 12.07.2007 US 929775 P
(43) Date of publication of application: 27.11.2013
(62) Divisional of application: 08780008.2
(73) Proprietor: The Brigham and Women's Hospital, Inc., Boston, MA 02115 (US)
(72) Inventor: Greenberg, Steven, Newton, MA Massachusetts 02458 (US)
(74) Representative: Jappy, John William Graham

(56) References cited:
- BAECHLER EMILY C ET AL: "An interferon signature in the peripheral blood of dermatomyositis patients is associated with disease activity", MOLECULAR MEDICINE (BALTIMORE), vol. 13, no. 1-2, January 2007 (2007-01), pages 59-68, XP008127184, ISSN: 1076-1551
- Emily Baechler: "An interferon signature in the peripheral blood of dermatomyositis patients is associated with disease activity", , 28 February 2007 (2007-02-28), pages 1-6, XP055082608, Retrieved from the Internet: URL:http://molmed.org/files/2069 [retrieved on 2013-10-04]
- O'CONNOR K A ET AL: "MxA gene expression in juvenile dermatomyositis peripheral blood mononuclear cells: Association with muscle involvement", CLINICAL IMMUNOLOGY, ACADEMIC PRESS, US, vol. 120, no. 3, 1 September 2006 (2006-09-01), pages 319-325, XP024941333, ISSN: 1521-6616, DOI: DOI:10.1016/J.CLIM.2006.05.011 [retrieved on 2006-09-01]
- ZHOU XIAODONG ET AL: "cDNA microarrays reveal distinct gene expression clusters in idiopathic inflammatory myopathies.", MEDICAL SCIENCE MONITOR : INTERNATIONAL MEDICAL JOURNAL OF EXPERIMENTAL AND CLINICAL RESEARCH JUL 2004 LNKD- PUBMED:15232492, vol. 10, no. 7, July 2004 (2004-07), pages BR191-BR197, XP009142028, ISSN: 1234-1010
- GREENBERG S A ET AL: "Molecular profiles of inflammatory myopathies", NEUROLOGY, LIPPINCOTT WILLIAMS & WILKINS, PHILADELPHIA, US, vol. 59, no. 8, 1 January 2002 (2002-01-01), pages 1170-1182, XP008127168, ISSN: 0028-3878
- TOUKAP A NZEUSSEU ET AL: "Identification of distinct gene expression profiles in the synovium of patients with systemic lupus erythematosus", ARTHRITIS & RHEUMATISM, vol. 56, no. 5, May 2007 (2007-05), pages 1579-1588, XP002612544, ISSN: 0004-3591
- RAJU RAGHAVAN ET AL: "Gene expression profile in the muscles of patients with inflammatory myopathies: effect of therapy with IVIg and biological validation of clinically relevant genes", BRAIN, vol. 128, no. Part 8, August 2005 (2005-08), pages 1887-1896, XP002612545, ISSN: 0006-8950
- BAECHLER EMILY C ET AL: "Gene expression profiling in human autoimmunity.", IMMUNOLOGICAL REVIEWS APR 2006 LNKD- PUBMED:16623768, vol. 210, April 2006 (2006-04), pages 120-137, XP002612546, ISSN: 0105-2896
- AMANDA L EVANS ET AL: "Generation and use of a tailored gene array to investigate vascular biology", ANGIOGENESIS, KLUWER ACADEMIC PUBLISHERS, DO, vol. 6, no. 2, 1 January 2004 (2004-01-01) , pages 93-104, XP019226838, ISSN: 1573-7209, DOI: DOI:10.1023/B:AGEN.0000011732.83724.E5

## Description

### Field of the Invention

The invention is in the field of methods that can be used to determine whether a subject exhibits a gene expression profile characteristic of inflammatory myopathies.

### Background of the Invention

Inflammatory myopathies are a group of diseases that involve inflammation of muscles or associated tissues and which are characterized primarily by weakness, muscle atrophy and, sometimes, pain. The three major subtypes of inflammatory myopathy are dermatomyositis (DM), polymyositis (PM), and inclusion body myositis (IBM). PM and DM are clinically similar except that DM is associated with skin rashes whereas PM is not. Although DM has been characterized as a disease caused by antibody attack of endothelial antigens (Dalakas, et al., Lancet 362(9388):971-82 (2003)), no well characterized pathogenic antibodies or endothelial antigens have been identified (Greenberg, et al., Curr. Opin. Neurol. 17(3):359-64 (2004)).

IBM is often mistakenly diagnosed as PM but, unlike PM or DM, typically does not respond to treatment with immunosuppressive drugs. Diagnosis of IBM is usually based upon biopsy results revealing muscle cells with inclusion bodies, *i.e*. with vacuoles containing amyloid protein. There currently is no effective treatment for IBM but patients may sometimes benefit from the administration of prednisone or intravenous immunoglobulin (IVIG).

Gene expression profiling of muscle in adult DM compared with other inflammatory myopathies and normal healthy controls has revealed a gene transcriptional signature that is dominated by the upregulation of interferon-α/β (IFN-α/β)-inducible genes Greenberg, et al., Ann. Neurol. 57(5):664-78 (2005)). Plasmacytoid dendritic cells (PDCs), natural IFN-α/βproducing cells, are present in DM muscle. The interferon-α/β induced protein, MxA, is expressed in perifascicular myofibers and capillaries. These observations suggest that tissue damage in DM derives from a self-destructive over-activation of the innate immune system (Greenberg, et al., Ann. Neurol. 57(5):664-78 (2005)). Further characterization of gene expression patterns in DM, PM and IBM may lead to better methods for distinguishing the inflammatory myopathies from one another and from other diseases characterized by progressive muscle weakness.

### Summary of the Invention

The present invention is based upon the quantitative measurement of blood RNA transcripts produced by the gene RSAD2. A high level of this transcript has high positive predictive value for a diagnosis of dermatomyositis or polymyositis, and excludes a diagnosis of the related inflammatory myopathy inclusion body myositis (IBM). High levels of transcripts for this gene are not seen in other muscle conditions, such as muscular dystrophy or myasthenia gravis, that may be important to distinguish diagnostically.

In its first aspect, the invention is directed to a method for determining whether a subject exhibits a gene expression pattern characteristic of polymyositis or dermatomyositis and not characteristic of inclusion body myositis (IBM), comprising (a) assaying a test biological sample of peripheral blood mononuclear cells for the expression of the gene radical S-adenosyl domain/CIG5 (RSAD2); and (b) concluding that said subject has a gene expression profile that is characteristic of dermatomyositis or polymyositis, and is not characteristic of IBM, if the results determined in the assaying indicate that the RSAD2 gene is expressed at least 10 fold higher in said test sample than in one or more control samples.

Preferably the method further comprises assaying the test sample for the expression of one or more additional genes selected from the group consisting of: interferon alpha-inducible protein 27; interferon-induced protein 44-like; interferon-induced protein 44; hypothetical protein LOC129607; 2',5'-oligoadenylate synthetase 1; XIAP-associated factor-1; interferon-induced tetratricopeptide 5; 2'-5'-oligoadenylate synthetase-like; 2'-5'-oligoadenylate synthetase 3; interferon-induced tetratricopeptide 1; phospholipid scramblase 1; hect domain and RLD 5; interferon-inducible protein kinase; TNF superfamily member 10; guanylate-binding protein 1; TNFalpha-induced protein 6; interferon-induced tetratricopeptide 3; sterile alpha motif domain 9-like; chromatin-modifying protein 5; ISG15 ubiquitin-like modifier; 2'-5'-oligoadenylate synthetase 2; interferon-induced helicase C domain 1; myxovirus resistance 1; and epithelial stromal interaction gene 1. Names of the genes, abbreviations for each and accession numbers for sequences in UniGene and GenBank databases are provided in Table 4.

For the purposes of the present invention, assays for determining the level of expression of a gene may be directed either at nucleic acids (*e.g*., using PCR amplification of mRNA) or at gene products (*e.g*., using an ELISA or radioimmunoassay). The results obtained using the test sample are compared with results from one or more control samples selected using criteria well known in the art. The control samples may be, for example, samples of blood, serum or plasma derived from individuals known to be free of an inflammatory myopathy or other muscle disease or they may be taken from the population as a whole and, optionally, matched with the test sample with respect to the age of the subject, sex, etc. By comparing the results from the controls and the test sample, a conclusion can be drawn with regard to whether the subject has an inflammatory myopathy and, if present, the particular type of inflammatory myopathy. The more genes exhibiting differences and the greater the magnitude of the differences, the greater the risk of a subject being positive for disease. Preferably assays will be performed on at least 5 genes and, more preferably, at least 15 genes. An increase of at least 10 fold in samples derived from subjects, compared to controls, should generally be observed in patients with an inflammatory myopathy with greater increases (20 or 50 fold) being more characteristic of DM or PM. The most preferred genes listed in Table 2 are those exhibiting the greatest difference in expression level in disease carrying individuals relative to controls.

In a preferred embodiment, the method of the invention is performed using a microarray plate or slide having a series of distinct, immobilized oligonucleotides recognizing the sequences of the genes listed above. The term "distinct" indicates that the oligonucleotides have different sequences that allow them to hybridize to different complementary sequences. Many methods are known in the art for producing plates or slides of this nature and any of these methods are compatible with the present invention. The plates or slides must include immobilized oligonucleotides that hybridize under stringent conditions to at least one of the genes set forth above, and, preferably, slides include several distinct oligonucleotides binding to different genes. The term "stringent conditions" indicates conditions that essentially only permit hybridization to occur with the exact complementary sequence of the immobilized oligonucleotide. In general, these hybridizations are performed in buffers of about neutral pH containing 0.1-0.5 NaCl and at a temperature of between 45 and 70°C. It is also possible to carry out incubations under conditions of low stringency and then to use high stringency wash conditions to cause the dissociation of hybridized sequences that are not exact matches. Procedures for carrying out incubations of this type in connection with microarray plates or slides are well known in the art.

Each group of immobilized oligonucleotides hybridizing to a specific gene will occupy a separate location on the microarray plate or slide and in total, there should be no more than 100 distinct oligonucleotides present. In preferred embodiments, there are at least 10 distinct oligonucleotides immobilized on plates that hybridize under stringent conditions to different genes with at least 15 such immobilized oligonucleotides being preferred. For economic reasons, it is also preferred that the total number of immobilized sequences present be 100 or less, more preferably, 50 or less.

The microarray plates described are preferably used in carrying out any of the methods of analyzing samples discussed herein. One way of carrying out an analysis would involve lysing cells and then amplifying the mRNA released in the presence of a detectable label, *e.g*., a nucleotide bound to a dye or other marker and present in a PCR primer. Thus, a population of labeled cDNAs is obtained that can be used directly in hybridizations with oligonucleotides immobilized on a microarray plate or slide. It is also possible to compare two different populations of mRNAs by carrying out PCR in the presence of different dyes for each population. After hybridizations are completed, plates are analyzed using an automated reader to determine the amount of label associated with each immobilized sequence, which reflects the abundance of the hybridized sequence in the original lysate. Many variations of this basic procedure have been described in the art and are compatible with the present invention.

### Detailed Description of the Invention

### I. Preparation of Samples for Use in Hybridizations

Samples containing peripheral blood mononuclear cells (PBMCs) may be obtained, lysed and extracted to obtain RNA using procedures described herein and that are standard in the art. Assays may be performed using standard procedures described by microarray plate manufactures and, in general, as described previously Greenberg, et al., Neurology 59(8):1170-82 (2002)). Alternatively, mRNA can be obtained from muscle cells obtained during biopsies and analyzed.

### II. Microarray Materials and Assays

All of the genes identified herein as being altered in polymyositis or dermatomyositis patients were present on the Affymetrix plates described in the Examples section. In principle, the same plates could be used for evaluating the mRNA from PBMCs or muscle cells. However, since the plates used include immobilized oligonucleotides for thousands of different genes, the system is inconvenient and unnecessarily expensive. Plates better suited to the analysis discussed herein can be made by focusing on the genes listed in Table 2. Thus, plates similar to the Affymetrix plates may be used, under the same assay conditions, but with only a small number of distinct hybridization sites (*e.g*., 5-50 or 5-100). This simplifies the analysis and allows for replicates to be included to better check on the consistency of results.

Although the same procedures and hardware described by Affymetrix could be employed in connection with the present invention, other alternatives are also available. Many reviews have been written detailing methods for making microarrays and for carrying out assays (see, *e.g*., Bowtell, Nature Genetics Suppl. 21:25-32 (1999); Constantine, et al., Life Sci. News 1:11-13 (1998); Ramsay, Nature Biotechnol. 16:40-44 (1998)). In addition, patents have issued describing techniques for producing microarray plates, slides and related instruments (U.S. 6,902,702; U.S. 6,594,432; U.S. 5,622,826) and for carrying out assays (U.S. 6,902,900; U.S. 6,759,197). The two main techniques for making plates or slides involve either polylithographic methods (see U.S. 5,445,934; U.S. 5,744,305) or robotic spotting methods (U.S. 5,807,522). Other procedures may involve inkjet printing or capillary spotting (see, *e.g*., WO 98/29736 or WO 00/01859).

The substrate used for microarray plates or slides can be any material capable of binding to and immobilizing oligonucleotides including plastic, metals such a platinum and glass. A preferred substrate is glass coated with a material that promotes oligonucleotide binding such as polylysine (see Chena, et al., Science 270:467-470 (1995)). Many schemes for covalently attaching oligonucleotides have been described and are suitable for use in connection with the method of the present invention (see, *e.g.,* U.S. 6,594,432). The immobilized oligonucleotides should be, at a minimum, 20 bases in length and should have a sequence exactly corresponding to a segment in the gene targeted for hybridization.

### III. Comments Regarding Additional Methodology

Although the methods described above may be used to determine the level of gene expression in PBMCs, any other procedure for conducting this analysis may also be used in connection with the invention. For example, DNA blotting techniques, with or without PCR amplification, may be used to quantitate levels of genes. Western blots or immunoassays may be used to quantitate gene products and, in some cases, enzyme-based assays may be used. The level of expression can also be assessed by immunofluorescence techniques or promoter based reporter assays. The essential element of the procedure is not how quantitation is performed, but rather the particular genes being examined and the determination of whether those genes are being expressed at a level characteristic of the presence or state of PM or DM, and not characteristic of IBM.

### V. Comments Regarding Utility

The assays described herein are designed to assess whether PBMCs have a gene expression profile characteristic of polymyositis or dermatomyositis, and not characteristic of inclusion body myositis. This is clearly of great value to scientists that are studying these diseases, and to clinicians trying to make a diagnosis or to determine whether a treatment regimen or drug is having a beneficial effect.

### Examples

Affymetrix whole genome microarrays were used to measure the expression of approximately 38,500 genes in 65 blood and 15 muscle samples from 56 subjects with dermatomyositis, polymyositis, inclusion body myositis, myasthenia gravis, muscular dystrophy and healthy volunteers. In addition, nine paired blood samples from the same individuals at different times with differing disease activity were compared. Bioinformatics techniques were used to identify genes with significant differential gene expression among diagnostic categories and in relationship to disease activity. The microarray data was corroborated with quantitative real-time PCR.

Most patients with active dermatomyositis and polymyositis, but not patients with inclusion body myositis, were found to have significant and high upregulation of the type 1 interferon-α/β-inducible genes in blood. Furthermore, the upregulation of these genes correlates with disease activity in dermatomyositis and polymyositis, with downregulation occurring when disease is controlled with treatment.

### Materials and Methods:

### Study subjects

65 microarray experiments were performed on blood samples from a total of 56 prospectively enrolled subjects of which 36 had inflammatory myopathies (12 with DM, 11 with PM, and 13 with IBM). For additional control groups, we studied five patients with myasthenia gravis, a non-inflammatory autoimmune myopathy, three patients with genetically determined myopathies (two with myotonic dystrophy type 2 and one with mitochondrial myopathy), and 12 healthy subject volunteers. Six patients with DM and two with PM provided second blood samples for microarray experiments performed at two different time points, one where there was active disease, the other when disease was improving; one patient with refractory DM provided two samples at different time points both when there was active disease. All patients met research criteria for definite or probable DM or PM (Hoogendijk, et al., Neuromuscul. Disord. 14(5):337-45 (2004)) and definite or possible IBM (Griggs, et al., Ann. Neurol. 38(5):705-13 (1995)). Patients with systemic lupus erythematosus were excluded. The clinical features of the patients with DM (mean age 47 years) and PM (mean age 54 years) are outlined in Table 1. Six patients, two with DM and four with PM, had interstitial lung disease. Of these, the two DM and one of the PM patients additionally had anti-histidyl transfer RNA (anti-Jo-1) antibodies. Patients with IBM, five men and seven women, had an average age of 69 years, and none were receiving immunomodulatory medication. Healthy volunteers, at the time of recruitment, had not had any serious illness in the last six months, had not started any new medications in the last six months and had no serious cold, flu or other infections in the previous two months. The volunteers were made up of five men and seven women and had an average age of 46 (range 30-62). An Internal Review Board approved the study. Written informed consent was obtained from all participating patients and healthy volunteers.

### Assessment of disease activity

We classified the DM and PM patients as those with active disease (DMA; PMA) and those with improving disease (DMI; PMI). Those patients who had 3 of the following 4 were classified as active: (1) increasing symptoms, (2) increasing objective weakness on manual muscle testing, (3) elevated and, if more than one measurement available, increasing serum creatine kinase (CK) level and (4) the treating physician increased the patient's immunotherapy. Similar features have been previously used to define active disease in myositis (Nagy, et al., Immunol. Lett. 74(3):207-10 (2000)). DM and PM patients were classified as active or improving prospectively, prior to analysis of gene expression data. Manual muscle testing using MRC grading was used to assess strength; a composite score for 30 different muscle groups was calculated, giving a maximum score of 150. We used the Myositis Intention to Treat Activity Index (MITAX) (Isenberg, et al., Rheumatology (Oxford) 43(1):49-54 (2004)), as proposed by the International Myositis Assessment and Clinical Studies group as an additional measure of disease activity. The MITAX is a multi-system assessment tool looking at the muscle, mucocutaneous, gastrointestinal, respiratory and musculoskeletal systems. Good inter-rater reliability has been reported for this measure of disease activity. In the nine patients with paired samples we saw an average reduction in MITAX score of 8.5 between active and improving. Active scores ranged from 12-13 and improving scores ranged from 2-6.

### PBMC collection, muscle tissue collection, and RNA extraction

We collected 10 mls of blood from patients and volunteers into EDTA-containing tubes (57 samples), or in some cases directly into PAX-Gene-RNA tubes (8 samples). For the EDTA-containing tubes, after centrifugation, we aspirated the plasma (upper layer) down to 1mm from the red blood cells, and then carefully aspirated 500 µl of buffy coat into Cryostat Storage Tubes, already filled with 1.2mls of solution of RNAlater (Ambion, Austin, Texas). We froze the combined buffy coat and RNAlater at -20°C. RNA was extracted using "Ribo Pure" (Ambion, Austin, Texas) from the buffy coat and from PAX-Gene RNA tubes. RNA concentration was measured using a spectrophotometer, and RNA quality was evaluated by running 1 µg of RNA on 1% agarose gels. Muscle biopsy samples weighing 70 to 120 mg had RNA extracted as previously described (Greenberg, et al., Neurology 59(8):1170-82 (2002)). Muscle biopsy tissue was obtained from 15 patients (5 DM, 5 PM, and 5 IBM) at the time of active disease, all of whom also had blood microarray studies at active or improving time points, and from 5 patients without neuromuscular disease undergoing diagnostic biopsies. Muscle RNA extraction was done with RiboPure similarly to PBMC RNA extraction. Of these 15 inflammatory myopathy muscle microarray studies, 9 (3 with DM, 2 with PM, and 4 with IBM) were previously performed with portions of the data used in publication, and reanalyzed in this study, and 6 were newly performed specifically for these studies.

### Target preparation, hybridization, and signal detection

Microarray studies were performed for muscle as previously described, using Affymetrix HG-U133A microarrays (Greenberg, et al., Neurology 59(8):1170-82 (2002)). PBMC samples were processed using Affymetrix HG-U133A plus 2.0 microarrays and GeneChip Operating System (GCOSv1.3) version 1.3.

### Data processing

The Affymetrix HG-U133 plus 2.0 GeneChip has 54,675 probe sets including 63 control probe sets. Probe set annotations were obtained from NetAffx Analysis Center, version 3/9/2007. The expression levels were calculated using GC-Content Robust Multichip Analysis (GCRMA), which was implemented in the Bioconductor *GCRMA* package (available at http://www.bioconductor.org/download/oldrelease/bioc1.6/popular /gcrma.html). This algorithm produces an improved expression measurement by accounting for GC-content based bias and optical noise behavior from all the arrays in an experiment (Wu, et al., J. Comput. Biol. 12(6):882-93 (2005)). Quality control was performed by visual inspection of scanned and reconstructed images to identify gross artifact and by careful assessment of the quality assessment parameters including control probe sets. All blood and muscle microarray data were analyzed together with GCRMA in this study.

### Data analysis and visualization

The average and 90% confidence intervals (CIs) of fold changes were calculated for each disease group compared to control group in addition to p-value of two group comparisons using Welch's t-test (Table 2). We applied stringent criteria to select genes as significantly upregulated, requiring a p-value < 0.0001, and the lower bound of CIs > 4.0. Genes were identified as IFN-α/β induced through searches of literature (10-12) and molecular databases.

Group fold changes and CIs were calculated comparing 8 DMA, 11 DMI, 7 PMA, 6 PMI, 13 IBM, 5 MG, 3 genetically determined myopathies, and 12 normal blood specimens. Additionally, 9 patients (7 with DM, 2 with PM) with paired samples (18 samples) were analyzed pair-wise for treatment associated changes in gene signatures. Blood and muscle expression data were compared for 13,398 genes common to both HG-U133A and HG-U133A plus 2.0 microarray chips mapped according to Affymetrix probeset identifications.

### Quantitative Real-Time Polymerase Chain Reaction (qRT-PCR)

We performed quantitative real-time PCR for two IFN-inducible genes: IFITI and MX1 on 18 samples (4 DMA, 5 DMI, 4 IBM, and 5 healthy volunteers) using primers designed with Primer software (Whitehead Institute, Cambridge, MA) and purchased commercially (Operon Biotechnologies, Inc. Huntsville, AL). Primers used were as follows: MxA: forward 5'-CGGCTAACGGATAAGCAGAG-3' (SEQ ID NO:1), and reverse 5'-ACCTACAGCTGGCTCCTGAA-3' (SEQ ID NO:2; IFIT1: forward 5'- AAAAGCCCAC-ATTTGAGGTG -3' (SEQ ID NO:3), and reverse 5'- GAAATTCCTGAAACCGACCA-3' (SEQ ID NO:4).

RNA (1 µg) was reverse-transcribed to cDNA with oligo(dT)20 and Ready-to-Go reverse transcription kit from (Amersham Biosciences, Piscataway, New Jersey). SYBR Green I-based real-time PCR was carried out on Opticon Monitor (MJ Research, Inc, Waltham, MA) with cDNA templates (1/100 of the RT reaction) using Promega (Madison, WI) taq polymerase and buffer, 2mM MgC12, 400 mM deoxy-NTP (Roche), 0.5X SYBR Green I, 0.8 mM of each PCR primer (Operon), in a 25 ml final volume reaction. The samples were loaded into wells of Low Profile 96-well microplates. After an initial denaturation step at 95°C for 5 min, conditions for cycling were 40 cycles of denaturation (95°C for 30s), annealing (for 30s), and extension (72°C for 1 min). The fluorescence signal was measured immediately after incubation at 79°C for 5s following each extension step, eliminating possible primer dimer detection. At the end of PCR cycles, a melting curve was generated to confirm the specificity of the PCR product. For each run, serial dilutions of human GAPDH plasmids were used as standards for quantitative measurement of the amount of amplified cDNA. All PCR reactions were run in triplicate. Comparative CT method was used to quantify the amplified transcripts. Mean fold ratios of amplified transcripts were calculated comparing DMI/DMA, DMA/Normal, DMI/Normal and IBM/Normal.

### Immunohistochemistry

Frozen muscle sections from 15 patients (5 each with DM, PM, and IBM) whose muscle underwent microarray studies were stained with antibodies against myxovirus resistance A (anti-MxA antibodies; courtesy of Dr. Otto Haller, Department of Virology, University of Freiburg, Germany) as previously described (Greenberg, et al., Ann. Neurol. 57(5):664-78 (2005)) and examined for correlation with transcript studies.

### Results

### Blood interferon-α/β-inducible gene transcripts are the most upregulated of all genes in PBMCs from patients with active DM and to a lesser extent active PM

Comparing transcript expression levels for active DM (DMA) and PM (PMA) with healthy controls, genes induced by interferon-α/β had the largest fold changes and highest statistical significance among the approximately 38,500 measured transcripts (p values < 0.0001) (Table 2). Of the 25 most highly upregulated genes, at least 21 (84%) are known to be interferon-α/β-inducible. None of these genes were significantly upregulated in patients with IBM, MG or genetically determined myopathies. The magnitude of upregulation was generally higher in DM than in PM. Quantitative RT-PCR showed that the interferon-inducible genes Mxl and IFIT1 were highly upregulated in DMA blood supporting our observations from the microarray data (Table 3). The average coefficient of variance of triplicate samples was 0.15, with a high correlation between triplicate runs of 0.99. Correlation of the RT-PCR data with microarray data was excellent (Mx1: R² = 0.9889; IFIT1: R² = 0.9978). Overall, 8 of 8 patients with DMA and 5 of 7 patients with PMA had levels of overexpression of interferon-α/β-inducible genes that exceeded that of all other 50 blood specimens studied.

### Interferon-α/β-inducible genes are downregulated with clinical improvement in DM and PM

We compared transcript profiles of 8 DMA samples with those of 11 DMI, and separately 7 PMA samples with 6 PMI. The genes most highly downregulated with improvement in disease are predominantly interferon-α/β-inducible (Table 2, right columns). Quantitative RT-PCR similarly confirmed improvement in DM patients (Table 3). In paired samples from the same patients with DM (N=6) or PM (N=2) who had active and improving disease at two different time points, the type 1 interferon-inducible genes were again the most downregulated of all genes. For the one patient with refractory DM, little overall change for many type 1 interferon-inducible genes was observed in the paired specimens.

### Upregulation interferon-α/β-inducible genes is greater in muscle than in blood in DM but not in PM or IBM

For 15 patients (5 each with DM, PM, and IBM), we compared the blood gene expression profiles with muscle gene expression using the 13,398 genes which are shared among both the U133A (used for muscle profiling) and U133 plus 2.0 (used for blood profiling) microarrays. In DM muscle, there is marked upregulation of the expression of the same interferon-α/β-inducible genes that we found to be highly upregulated in blood. In contrast, in PM and IBM muscle only a modest increase of the interferon-α/β-inducible gene transcription was present. This may have been due to infiltrating immune system cells that themselves express interferon-α/β-inducible genes, such as MxA. Of particular interest in DM, there is a marked overexpression of certain interferon-inducible genes in muscle in comparison to blood. For example, DM muscle expression of ISG15 was approximately 570 times that of normal muscle and about 100 fold higher than in DM blood.

### Upregulation of interferon-α/β-inducible MxA protein correlates with tissue pathology

As previously reported, the overexpression of one interferon-α/β-inducible gene protein, MxA, is present in muscle in DM (Greenberg, et al., Ann. Neurol. 57(5):664-78 (2005)). In the current study, MxA transcript level, although similarly elevated in DMA-blood (6.2 fold) and PMA-blood (6.0 fold), was markedly higher by microarray studies in DM-muscle (281 fold) compared to PM-muscle (2.5 fold). The marked enrichment of MxA transcript in DM muscle is similarly accompanied by marked enrichment of MxA protein by immunohistochemistry in comparing muscle sections from DM and PM. In 4 of 5 DM patients, MxA staining was present intensely in many myofibers, particularly perifascicular myofibers, while in all 5 patients with PM and 5 with IBM, MxA staining was limited to infiltrating immune system cells. MxA staining is not present in normal muscle biopsies.

### Discussion

Our findings suggest that, in most patients with DM and PM, but not in patients with IBM, there is a distinct blood gene expression profile characterized by marked overexpression of interferon-α/β-inducible genes. Clinical improvement during immunosuppressive treatment is generally associated with reduction in the overexpression of these genes towards normal levels. These findings, in relationship to gene expression in muscle, have implications for pathogenic hypotheses and blood biomarkers of potential diagnostic use.

For dermatomyositis, the interferon-α/β gene signature in blood is highly correlated with the findings of microarray studies in muscle and supports the hypothesis that this disease may be driven by systemic and intramuscular overproduction of interferon-α/β. Similar blood gene transcription signatures have been reported in systemic lupus erythematosus (SLE) (Baechler, et al., Proc. Nat'l Acad. Sci. USA 100(5):2610-5 (2003); Bennett, et al., J. Exp. Med. 197(6):711-23 (2003); Han, et al., Genes Immun. 4(3):177-86 (2003)). Overexpression at the protein level for at least one of these genes (MxA) is present in DM muscle capillaries and perifascicular myofibers, and DM skin (Wenzel, et al., Br. J. Dermatol. 153(2):462-3 and 463-4 (2005); Wenzel, et al., Clin. Exp. Dermatol. 31(4):576-82 (2006)). Additionally plasmacytoid dendritic cells (pDCs), natural IFN-α producing cells, are abundant in DM muscle (Greenberg, et al., Ann. Neurol. 57(5):664-78 (2005)) and skin (Wenzel, et al., Clin. Exp. Dermatol. 31(4):576-82 (2006)). Upregulation of MxA transcript levels in blood have been observed in juvenile DM and may correlate with disease activity (O'Connor, et al., Clin. Immunol. 120(3):319-25 (2006)).

Although blood profiles exhibited similar levels of overexpression of interferon-α/β-inducible genes in both DM and PM, in muscle some of these genes are orders of magnitude more highly expressed only in DM. One explanation for this could be that although systemic activation of the innate immune system is present in both diseases, DM muscle is exposed to a greater amount of type 1 interferons than PM muscle. This hypothesis is supported by previous findings of interferon-α/β secreting plasmacytoid dendritic cells infiltrating DM muscle (Greenberg, et al., Ann. Neurol. 57(5):664-78 (2005)) in much greater numbers than seen in IBM and PM (Greenberg, et al., Muscle Nerve 35(1):17-23 (2007)). Additionally, whereas in PM the expression of the interferon-α/β-inducible protein MxA is confined to invading inflammatory cells, in DM MxA protein is present within myofibers.

The enrichment of such specific interferon-α/β-inducible genes in muscle likely is an important clue to the nature of tissue injury in DM. Thus, the marked enrichment of ISG15 transcript in DM muscle suggests that of the various interferon-α/β-inducible proteins upregulated in DM blood and muscle, this particular molecule, a ubiquitin-like modifier, could be of greater relevance to the direct mechanisms of tissue injury in DM.

The distinct lack of highly upregulated interferon-α/β genes in IBM blood, compared with PM blood, contrasts with the otherwise similar nature of immunological abnormalities that have previously been observed in muscle in these two diseases. These findings suggest a different magnitude of activation of the innate immune system in PM than IBM. Further study of this hypothesis would best be addressed in larger numbers of patients. Additionally, for many patients the diagnosis of IBM is delayed, recognized only after a previous diagnosis of glucocorticoid-resistant PM (Amato, et al., Ann. Neurol. 40(4):581-6 (1996)). Further characterization of the interferon-α/β-inducible gene blood biomarkers in IBM and PM suggests the potential for future earlier diagnosis of IBM and avoidance of glucocorticoid treatment for such patients.

Our findings also suggest the utility of blood biomarkers of disease activity to supplement management of patients with DM and PM. In this study, we have identified multiple blood biomarkers of active medication responsive myositis. Currently there is a need for more specific tests to evaluate disease activity in DM or PM. The level of serum creatine kinase (CK) is generally reflective of disease activity in PM, but may be normal in patients with active DM. The MITAX has been proposed as a clinical measure of disease activity. We calculated a MITAX score for our DM patients, which correlated well with our own assessment of disease activity. However, while the MITAX has been shown to be a good tool for disease activity assessment, intra-class correlation between assessors for muscle involvement was low, underlining the need for a more objective measure (Isenberg, et al., Rheumatology (Oxford) 43(1):49-54 (2004)). An objective and inexpensive PCR based blood test which correlates the expression of certain interferon-α/β-inducible genes with disease activity could supplement the clinical management of DM and PM. Eventually such tests might provide surrogate markers for treatment response in clinical trials.

**Table 1. Clinical features of 23 patients with dermatomyositis and polymyositis.**

| Paired blood specimens were studied from those 9 patients marked with an asterisk (*) for a total of 32 blood samples from patients with DM and PM. | | | | | | | | | | | | |
|---|---|---|---|---|---|---|---|---|---|---|---|---|
| **Patient ID/ Disease** | **A/S** | **PA** | **DD** | **Treatment (Tx)** | **Tx-D** | **CK** | **S** | **MMT score** | **X** | **Y** | **Z** | **Other Diagnoses** |
| | | | | | | | | | | | | |

| **Dermatomyositis (DM) N=12** | | | | | | | | | | | | |
|---|---|---|---|---|---|---|---|---|---|---|---|---|
| BGE10-DMA* | 38/F | No | 108 | Pred/IVIG | 4 | 948 | ↑ | 142 | ↓ | ↑ | 12 | - |
| BGE19-DMA* | 61/F | Yes | 12 | Pred/Myco | 24 | 165 | ↑ | 139 | ↓ | ↑ | 13 | Breast ca |
| BGE46-DMA* | 25/F | Yes | 8 | Pred | 0.1 | 393 | ↑ | 128 | ↓ | ↑ | 12 | - |
| BGE79-DMA* | 46/F | No | 72 | None | 0 | 740 | ↑ | 133 | ↓ | ↑ | 12 | Calcinosis |
| BGE92-DMA* | 27/F | Yes | 17 | Pred | 11 | 1140 | ↑ | 146 | ↓ | ↑ | 13 | ILD, Jo-1 |
| BGE95-DMA* | 53/M | Yes | 25 | MTX | 23 | 6416 | ↑ | 131 | ↓ | ↑ | 13 | Diabetes |
| BGE99-DMA* | 21/M | Yes | 3 | None | 0 | 352 | ↑ | 148 | ↓ | ↑ | 12 | - |
| BGE110-DMA | 54/F | Yes | 6 | Pred | 2 | 1140 | ↑ | 146 | ↓ | ↑ | 13 | ILD, Jo-1 |
| BGE15-DMI | 62/F | Yes | 36 | Pred/Myco | 12 | 80 | ↓ | 150 | f | ↓ | 2 | |
| BGE17-DMI | 70/F | No | 24 | Pred/IVIG/ Myco | 6 | 19 | ↓ | 123 | ↑ | ↓ | 2 | - |
| BGE36-DMI | 59/F | No | 12 | Pred/Myco | 1 | 1652 | ↓ | 140 | ↓ | ↓ | 2 | - |
| BGE80-DMI | 44/F | No | 1 | Pred | 1 | 71 | ↓ | 140 | ↓ | ↓ | 2 | - |
| | | | | | | | | | | | | |

| **Polymyositis (PM) N=11** | | | | | | | | | | | | |
|---|---|---|---|---|---|---|---|---|---|---|---|---|
| BGE3-PMA | 55/F | No | 26 | Pred/Myco | 20 | 2100 | ↑ | 140 | ↓ | ↑ | na | - |
| BGE32-PMA | 72/F | No | 3 | Pred/IVIG | 2 | 1219 | ↑ | 100 | ↓ | ↑ | na | - |
| BGE47-PMA | 72/F | No | 11 | Pred/Myco | 9 | 3027 | ↑ | 118 | ↓ | ↑ | na | - |
| BGE98-PMA | 38/F | No | 16 | Pred | 16 | 129 | ↑ | 139.66 | ↓ | ↑ | na | MCTD |
| BGE106-PMA* | 59/F | No | 2 | None | 0 | 4720 | ↑ | 128.65 | ↓ | ↑ | na | - |
| BGE119-PMA* | 47/F | No | 5 | None | 0 | 1256 | ↑ | 135 | ↓ | ↑ | na | ILD/MCTD |
| BGE121-PMA | 67/M | No | 12 | None | 0 | 1102 | ↑ | 137 | ↓ | ↑ | na | ILD |
| BGE11-PMI | 65/F | No | 81 | Aza/IVIG | 48 | 472 | ↓ | 149 | ↑ | ↓ | na | ILD, Jo-1 |
| BGE26-PMI | 71/F | No | 29 | Pred/Aza/ IVIG | 29 | 29 | ↓ | 149 | ↑ | ↓ | na | ILD |
| BGE50-PMI | 40/F | No | 2 | Pred/IVIG | 2 | 663 | ↓ | 138 | ↑ | ↓ | na | - |
| BGE58-PMI | 30/M | No | 25 | Pred/Mtx/ IVIG | 25 | 1743 | ↓ | 119 | ↑ | ↓ | na | - |

| | | | | | | | | | | | | |
|---|---|---|---|---|---|---|---|---|---|---|---|---|
| A/S=Age/sex; AZA=azathioprine; CK=creatine kinase; DD=Disease duration; DMA=DM active; DMI=DM improving; DMS=DM sine myositis; f=full; IVIG=intravenous immunoglobulin; MCTD=mixed connective tissue disease; ILD=interstitial lung disease; Meds=medications; MMT=manual muscle testing; PA= Perifascicular atrophy; Δ=change; PMA=PM active; PMI=PM improving; Pred=prednisone; Myco=mycophenolate mofetil; MTX=methotrexate; S=symptoms; MITAX=Myositis Intention to Treat Activity Index; Tx-D=Disease duration (months); X=Δ MMTscore; Y=Δ Meds; Z=MITAX score (DM only) | | | | | | | | | | | | |

**Table 2. Interferon-α/β-inducible genes are the most highly overexpressed genes in active dermatomyositis and polymyositis, but not other control groups.**

| The highest differentially expressed genes are listed in descending order of fold-ratios for active DM (DMA) compared to normal. The gene symbols of well-established interferon-α/β inducible genes are in bold. The marked upregulation in DMA (N=8) is also seen in active PM (PMA; N=7), but not active IBM (N=13), myasthenia gravis (N=5), or genetic myopathies (N=3) compared to normal (Norm; N=12). Criteria for gene significance was a p-value < 0.0001, with the top 25 genes ordered by fold change (with confidence intervals) listed. Comparison of Improving to Active disease groups show downregulation that occurs with treatment related improvement. | | | | | | | | | |
|---|---|---|---|---|---|---|---|---|---|
| **Gene Symbol** | **Gene Title** | **Disease Group Comparisons** | | | | | | **Improving/ Active Comparison** | |
| | | A | B | C | D | E | F | G | H |
| IFI27 | interferon alpha-inducible prt 27 | 7.94 E-06 | 130 (28-277) | 21.65 | 2.79 | 1.01 | 0.94 | -2.34 | 1.31 |
| IFI44L | interferon-induced prt 44-like | 2.84 E-07 | 104 (39-194) | 64.23 | 7.01 | 0.68 | 4.09 | -9.65 | -5.16 |
| RSAD2 | radical S-adenosyl domain/CIG5 | 2.20 E-07 | 66 (26-154) | 42.04 | 4.63 | 1.09 | 2.98 | -9.76 | -6.00 |
| IFI44 | interferon-induced prt 44 | 1.61 E-06 | 57 (17-104) | 27.28 | 2.94 | 0.52 | 1.46 | -21.26 | -7.11 |
| LOC129607 | hypothetical prt LOC129607 | 4.74 E-09 | 47 (20-114) | 29.34 | 3.48 | 1.20 | 2.51 | -16.42 | -5.68 |
| OASI | 2',5'-oligoadenylate synthetase 1 | 3.80 E-09 | 36 (13-84) | 15.13 | 2.05 | 0.35 | 1.04 | -18.22 | -3.90 |
| EPSTI1 | epithelial stromal interaction 1 | 4.27 E-07 | 33 (15-57) | 16.24 | 3.09 | 0.73 | 3.50 | -17.93 | -2.21 |
| BIRC4BP | XIAP associated factor-1 | 7.99 E-06 | 24 (12-39) | 17.38 | 4.18 | 0.78 | 1.76 | -13.25 | -3.45 |
| IFIT5 | interferon-induced tetratricopeptide 5 | 6.81 E-06 | 20 (10-32) | 14.65 | 2.63 | 0.89 | 1.98 | -13.24 | -3.65 |
| OASL | 2',5'-oligoadenylate synthetase-like | 3.23 E-08 | 19 (9-50) | 9.49 | 2.42 | 0.38 | 0.84 | -11.07 | -3.92 |
| OAS3 | 2',5'-oligoadenylate synthetase 3 | 7.74 E-06 | 19 (8-34) | 12.68 | 1.94 | 0.60 | 1.38 | -13.18 | -5.02 |
| IFIT1 | interferon-induced tetratricopeptide 1 | 2.87 E-06 | 18 (8-47) | 15.50 | 1.92 | 0.37 | 1.45 | -8.93 | -7.83 |
| PLSCR1 | phospholipid scramblase 1 | 5.49 E-07 | 16 (9-25) | 14.97 | 3.57 | 5.68 | 2.69 | -4.19 | -2.35 |
| HERC5 | hect domain and RLD 5 | 1.21 E-06 | 15 (5-30) | 11.62 | 1.91 | 0.49 | 1.13 | -13.58 | -4.14 |
| EIF2AK2 | interferon-inducible prt kinase | 2.78 E-06 | 13 (7-24) | 12.18 | 4.59 | 1.52 | 3.16 | -6.23 | -1.76 |
| TNFSF10 | TNF superfamily, member 10 | 6.42 E-05 | 13 (7-21) | 10.79 | 3.58 | 2.69 | 3.26 | -5.21 | -2.56 |
| GBP1 | guanylate binding prt 1 | 3.26 E-05 | 13 (6-21) | 6.73 | 3.83 | 0.54 | 3.32 | -9.79 | -1.75 |
| TNFAIP6 | TNF, alpha-induced prt 6 | 4.83 E-06 | 11 (6-17) | 8.05 | 1.76 | 3.06 | 1.65 | -4.44 | -2.00 |
| IFIT3 | interferon-induced tetratricopeptide 3 | 5.46 E-08 | 9 (6-14) | 7.41 | 1.84 | 0.59 | 1.82 | -5.45 | -2.95 |
| SAMD9L | sterile alpha motif domain 9-like | 1.59 E-05 | 9 (5-17 | 13.96 | 7.58 | 0.39 | 2.10 | -13.71 | 1.31 |
| CHMP5 | chromatin modifying protein 5 | 4.21 E-05 | 9 (5-13) | 6.42 | 1.60 | 1.90 | 1.88 | -4.21 | -2.44 |
| ISG15 | ISG15 ubiquitin-like modifier | 5.24 E-05 | 9 (4-15) | 4.86 | 1.04 | 0.19 | 0.55 | -5.05 | -4.62 |
| OAS2 | 2',5'-oligoadenylate synthetase 2 | 5.94 E-05 | 8 (4-14) | 4.15 | 0.94 | 0.38 | 1.23 | -9.74 | -6.46 |
| IFIH1 | interferon induced helicase C domain 1 | 1.50 E-05 | 7 (4-10) | 7.40 | 1.86 | 0.50 | 1.74 | -8.09 | -2.97 |
| MX1 | myxovirus resistance 1 | 5.21 E-06 | 6 (3-13) | 6.04 | 1.69 | 0.21 | 0.83 | -4.54 | -4.65 |

| | | | | | | | | | |
|---|---|---|---|---|---|---|---|---|---|
| prt=protein; A=*p*-value DMA/norm; B=DMA/norm Fold (confidence interval); C=PMA/norm Fold; D=IBM/norm Fold; E=MG/norm Fold; F=DYS/norm Fold; | | | | | | | | | |

**Table 3. Quantitative Reverse Transcriptase PCR of transcripts Mx1 and IFIT1**

| Transcripts were in blood from active DM (DMA; N=4), improving DM (DMI; N=5), IBM (N=4) and healthy volunteers (N=5); fold ratios are listed. Transcripts are upregulated in DM compared to normals and other inflammatory myopathies. Correlation of RT-PCR with microarray data was excellent (Mxl: R² = 0.9889; IFIT1: R² = 0.9978). | | | | |
|---|---|---|---|---|
| **Gene Symbol** | **DMI/DMA** | **DMA/Norm** | **DMI/Norm** | **IBM/Norm** |
| IFITI-array | -468 | 15.87 | 1.18 | 0.77 |
| IFITI-rtPCR | -9.31 | 55.55 | 5.97 | 1.77 |
| Mx1-array | -5.47 | 5.93 | 1.97 | 1.29 |
| Mx1-rtPCR | -4.45 | 25.68 | 5.77 | 1.38 |

**Table 4: List of Blood Gene RNA Transcripts**

| Genes identified by unique National Center for Bioinformatics UniGene ID number, which represents all sequences within the GenBank database; a single GenBank ID number is provided for an example of a representative sequence. | | | |
|---|---|---|---|
| **Gene Symbol** | **Gene Name** | **UniGene ID** | **GenBank ID** |
| IFI27 | interferon alpha-inducible protein 27 | Hs.532634 | BT006781.1 |
| IFI44 | interferon-induced protein 44 | Hs.82316 | NM 006417.4 |
| IFI44L | interferon-induced protein 44-like | Hs.389724 | NM 006820.2 |
| CMPK2 | Cytidine monophosphae kinase | Hs.7155 | NM 207315.2 |
| EIF2AK2 | Interferon-inducible protein kinase | Hs.131431 | AY302136.1 |
| EPSTI1 | epithelial stromal interaction 1 | Hs.546467 | AF396928.1 |
| BIRC4BP | XIAP associated factor-1 | Hs.441975 | BC058017.1 |
| OAS1 | 2',5'-oligoadenylate synthetase 1 | Hs.524760 | BT006785.1 |
| OAS2 | 2'-5'-oligoadenylate synthetase 2 | Hs.414332 | BC049215.1 |
| OAS3 | 2'-5'-oligoadenylate synthetase 3 | Hs.528634 | NM 006187.2 |
| OASL | 2'-5'-oligoadenylate synthetase-like | Hs.118633 | AJ225089.1 |
| IFIT1 | interferon-induced tetratricopeptide 1 | Hs.20315 | BT006667.1 |
| IFIT2 | interferon-induced tetratricopeptide 2 | Hs.437609 | AK312831.1 |
| IFIT3 | interferon-induced tetratricopeptide 3 | Hs.714337 | BT007284.1 |
| IFIT5 | interferon-induced tetratricopeptide 5 | Hs.252839 | NM 012420.1 |
| PLSCR1 | phospholipid scramblase 1 | Hs.130759 | AF098642.1 |
| HERC5 | hect domain and RLD 5 | Hs.26663 | AB027289.1 |
| GBP1 | guanylate binding protein 1 | Hs.62661 | BT006847.1 |
| MX1 | myxovirus resistance 1 | Hs.517307 | M30817.1 |
| RSAD2 | radical S-adenosyl domain / CIG5 | Hs.17518 | BC017969.1 |
| SAMD9L | Sterile alpha motif domain 9-like | Hs.489118 | BC127118.1 |
| TNFAIP6 | TNF, alpha-induced protein 6 | Hs.437322 | NM_007115.2 |
| TNFSF10 | TNF superfamily, member 10 | Hs.478275 | NM_003810.2 |
| CHMP5 | chromatin modifying protein 5 | Hs.635313 | AF229832.1 |
| ISG15 | ISG15 ubiquitin-like modifier | Hs.458485 | M13755.1 |
| IFIH1 | interferon induced helicase C domain 1 | Hs.163173 | BC046208.1 |

## Claims

1. A method of determining whether a subject exhibits a gene expression pattern characteristic of dermatomyositis or polymyositis, and not characteristic of inclusion body myositis (IBM), comprising:
a) assaying a test biological sample from said subject containing peripheral blood mononuclear cells, for the expression of the gene radical S-adenosyl domain/CIG5 (RSAD2);
b) concluding that said subject has a gene expression profile that is characteristic of dermatomyositis or polymyositis, and is not characteristic of IBM, if the results determined in the assaying indicate that the RSAD2 gene is expressed at least 10 fold higher in said test sample than in one or more control samples.

2. The method of claim 1, wherein said biological sample is a sample of blood, plasma or serum.

3. The method of any preceding claim, wherein said assaying is performed using a microarray plate.

4. The method of any preceding claim, further comprising assaying the test sample for the expression of one or more additional genes selected from the group consisting of: interferon alpha-inducible protein 27, interferon-induced protein 44-like, interferon-induced protein 44; hypothetical protein LOC129607; 2',5'-oligoadenylate synthetase 1; XIAP-associated factor-1; interferon-induced tetratricopeptide 5; 2'-5'-oligoadenylate synthetase-like; 2'-5'-oligoadenylate synthetase 3; interferon-induced tetratricopeptide 1; phospholipid scramblase 1; hect domain and RLD 5; interferon-inducible protein kinase; TNF superfamily, member 10; guanylate-binding protein 1; TNFalpha-induced protein 6; interferon-induced tetratricopeptide 3; sterile alpha motif domain 9-like; chromatin-modifying protein 5; ISG15 ubiquitin-like modifier; 2'-5'-oligoadenylate synthetase 2; interferon-induced helicase C domain 1; myxovirus resistance 1; and epithelial stromal interaction gene 1.

5. The method of claim 4, wherein at least 5 genes are assayed.

6. The method of claim 5, wherein at least 15 genes are assayed.

7. The method of any preceding claim, wherein the assay is performed using a microarray plate comprising a series of distinct immobilized oligonucleotides, wherein:
a) said oligonucleotides hybridize under stringent conditions specifically to gene sequence of the gene defined in claim 1;
b) said oligonucleotides are immobilized at a location on said microarray plate that does not contain any oligonucleotides that hybridize to other sequences under stringent conditions; and
c) said microarray plate comprises no more than 100 distinct immobilized oligonucleotides in total.

8. The method of claim 7, wherein said microarray plate additionally comprises oligonucleotides that hybridize under stringent conditions specifically to gene sequences of one or more of interferon alpha-inducible protein 27; interferon-induced protein 44-like; interferon-induced protein 44; hypothetical protein LOC129607; 2',5'-oligoadenylate synthetase 1; XIAP-associated factor-1; interferon-induced tetratricopeptide 5; 2'-5'-oligoadenylate synthetase-like; 2'-5'-oligoadenylate synthetase 3; interferon-induced tetratricopeptide 1; phospholipid scramblase 1; hect domain and RLD 5; interferon-inducible protein kinase; TNF superfamily, member 10; guanylate-binding protein 1; TNFalpha-induced protein 6; interferon-induced tetratricopeptide 3; sterile alpha motif domain 9-like; chromatin-modifying protein 5; ISG15 ubiquitin-like modifier; 2'-5'-oligoadenylate synthetase 2; interferon-induced helicase C domain 1; myxovirus resistance 1; and epithelial stromal interaction gene 1.

9. The method of claim 8, wherein said microarray plate comprises at least 10 of the distinct immobilized oligonucleotides, preferably at least 15 of the distinct immobilized oligonucleotides, and preferably which comprises distinct immobilized oligonucleotides that hybridize under stringent conditions specifically to all of said genes.

10. The method of any of claims 6 to 9, wherein said microarray plate comprises no more than 50 distinct immobilized oligonucleotides in total.

11. The method of any of claims 6 to 10, wherein said microarray plate does not comprise any distinct immobilized oligonucleotides that hybridize under stringent conditions to a gene other than a said gene.

12. The method of any preceding claim, wherein if the result determined in the assaying step indicates that the RSAD2 gene is expressed at least 20 fold higher in said test sample than in one or more control samples then it can be concluded that said subject has a gene expression profile that is characteristic of dermatomyositis or polymyositis and is not characteristic of IBM.

## Patentansprüche

1. Ein Verfahren zur Bestimmung, ob ein Patient ein Merkmal für ein Genexpressionsmuster für Dermatomyositis oder Polymyositis und kein Merkmal für Einschlusskörpermyositis (IBM) aufweist, bestehend aus:
a) Untersuchung einer biologischen Testprobe des genannten Patienten, die periphere mononukleäre Blutzellen enthält, zwecks Expression des Gens Radical S-Adenosyl Domain/CIG5 (RSAD2);
b) Feststellung, dass der genannte Patient ein Genexpressionsprofil aufweist, das für Dermatomyositis oder Polymyositis charakteristisch ist und nicht für IBM charakteristisch ist, wenn die durch die Untersuchung bestimmten Ergebnisse anzeigen, dass das RSAD2-Gen in der genannten Testprobe zumindest 10-fach höher exprimiert wird als einer oder mehreren Kontrollproben.

2. Das Verfahren entsprechend Anspruch 1, wobei es sich bei der biologischen Probe um eine Blut-, Plasma- oder Serumprobe handelt.

3. Das Verfahren entsprechend einem der vorhergehenden Ansprüche, wobei die Untersuchung unter Verwendung einer Microarray-Platte durchgeführt wird.

4. Das Verfahren entsprechend einem der vorhergehenden Ansprüche, zu dem weiterhin die Untersuchung der Testprobe auf Expression eines oder mehrerer weiterer Gene gehört, der/die aus der Gruppe bestehend aus Folgenden ausgewählt wird/werden: Interferon alpha-induzierbares Protein 27; Interferon-induziertes Protein 44-like; Interferon-induziertes Protein 44; hypothetisches Protein LOC129607; 2',5'-Oligoadenylat-Synthetase 1; XIAP-assoziierter Faktor-1; Interferon-induziertes Tetratricopeptid 5; 2'-5'-Oligoadenylat- Synthetase-like; 2'-5'-Oligoadenylat-Synthetase 3; Interferon-induziertes Tetratricopeptid 1; Phospholipid-Scramblase 1; Hect-Domäne und RLD 5; Interferon-induzierbare Proteinkinase; TNF-Superfamilien-Mitglied 10; Guanylat-bindendes Protein 1; TNF-alpha-induziertes Protein 6; Interferon-induziertes Tetratricopeptid 3; Sterile Alpha Motif Domain 9-like; Chromatin-modifizierendes Protein 5; ISG15 Ubiquitin-ähnlicher Modifikator; 2'-5'-Oligoadenylat-Synthetase 2; Interferon-induzierte Helicase-C-Domäne 1; Myxovirus-Resistenz 1 und epitheliales Stroma-Interaktions-Gen 1.

5. Das Verfahren entsprechend Anspruch 4, wobei zumindest 5 Gene getestet werden.

6. Das Verfahren entsprechend Anspruch 5, wobei zumindest 15 Gene getestet werden.

7. Das Verfahren entsprechend einem der vorhergehenden Ansprüche, wobei die Untersuchung unter Verwendung einer Microarray-Platte mit einer Reihe distinktiver immobilisierter Oligonukleotide durchgeführt wird, wobei:
a) die genannten Oligonukleotide unter strengen Bedingungen spezifisch zur Gensequenz des in Anspruch 1 definierten Gens hybridisieren;
b) die genannten Oligonukleotide an einer Stelle der genannten Microarray-Platte immobilisiert werden, die keine Oligonukleotide enthält, die unter strengen Bedingungen zu anderen Sequenzen hybridisieren; und
c) die genannte Microarray-Platte nicht mehr als insgesamt 100 distinktive immobilisierte Oligonukleotide enthält.

8. Das Verfahren entsprechend Anspruch 7, wobei die genannte Microarray-Platte zudem Oligonukleotide enthält, die unter strengen Bedingungen spezifisch zu Gensequenzen eines oder mehrerer der Folgenden hybridisieren, nämlich Interferon alpha-induzierbares Protein 27; Interferon-induziertes Protein 44-like; Interferon-induziertes Protein 44; hypothetisches Protein LOC129607; 2',5'-Oligoadenylat-Synthetase 1; XIAP-assoziierter Faktor-1; Interferon-induziertes Tetratricopeptid 5; 2'-5'-Oligoadenylat- Synthetase-like; 2'-5'-Oligoadenylat-Synthetase 3; Interferon-induziertes Tetratricopeptid 1; Phospholipid-Scramblase 1; Hect-Domäne und RLD 5; Interferon-induzierbare Proteinkinase; TNF-Superfamilien-Mitglied 10; Guanylat-bindendes Protein 1; TNF-alpha-induziertes Protein 6; Interferon-induziertes Tetratricopeptid 3; Sterile Alpha Motif Domain 9-like; Chromatin-modifizierendes Protein 5; ISG15 Ubiquitin-ähnlicher Modifikator; 2'-5'-Oligoadenylat-Synthetase 2; Interferon-induzierte Helicase-C-Domäne 1; Myxovirus-Resistenz 1 und epitheliales Stroma-Interaktions-Gen 1.

9. Das Verfahren entsprechend Anspruch 8, wobei die genannte Microarray-Platte zumindest 10 der distinktiven immobilisierten Oligonukleotide, bevorzugt zumindest 15 der distinktiven immobilisierten Oligonukleotide umfasst und diese bevorzugt distinktive immobilisierte Oligonukleotide umfassen, die unter strengen Bedingungen spezifisch zu allen der genannten Gene hybridisieren.

10. Das Verfahren entsprechend einem der Ansprüche 6 bis 9, wobei die genannte Microarray-Platte insgesamt nicht mehr als 50 distinktive immobilisierte Oligonukleotide enthält.

11. Das Verfahren entsprechend einem der Ansprüche 6 bis 10, wobei die genannte Microarray-Platte keine distinktiven immobilisierten Oligonukleotide umfasst, die unter strengen Bedingungen zu einem anderen als dem genannten Gen hybridisieren.

12. Das Verfahren entsprechend einem der vorhergehenden Ansprüche, wobei, wenn das im Testschritt bestimmte Ergebnis anzeigt, dass das RSAD2-Gen in der genannten Testpobe zumindest 20-fach höher als in einer oder mehreren Kontrollproben exprimiert wird, gefolgert werden kann, dass der genannte Patient ein Genexpressionsprofil aufweist, das für Dermatomyositis oder Polymyositis charakteristisch ist und nicht für IBM charakteristisch ist.

## Revendications

1. Procédé permettant de déterminer si un sujet manifeste une voie d'expression génique caractéristique de la dermatomyosite ou de la polymyosite et non pas caractéristique de la myosite à inclusions (IBM), comprenant les étapes consistant à :
a) procéder au dosage d'un échantillon biologique dudit sujet contenant des cellules mononucléaires de sang périphérique pour l'expression du radical génique appartenant au domaine S-adénosyl/CIG5 (RSAD2) ;
b) conclure que ledit sujet possède un profil d'expression génique qui est caractéristique de la dermatomyosite ou de la polymyosite et qui n'est pas caractéristique de l'IBM, si les résultats déterminés dans le dosage indiquent que le gène RSAD2 est exprimé à des niveaux au moins 10 fois plus élevés dans ledit échantillon de test que dans un ou plusieurs des échantillons témoins.

2. Procédé selon la revendication 1, dans lequel ledit échantillon biologique est un échantillon de sang, de plasma ou de sérum.

3. Procédé selon l'une quelconque des revendications précédentes, dans lequel ledit dosage est réalisé en utilisant une plaque de microréseaux.

4. Procédé selon l'une quelconque des revendications précédentes, comprenant en outre le dosage de l'échantillon de test pour l'expression d'un ou de plusieurs gènes supplémentaires sélectionnés dans le groupe constitué de : la protéine 27 inductible par l'interféron alpha, la protéine de type 44 induite par l'interféron, la protéine 44 induite par l'interféron ; la protéine LOC129607 hypothétique ; la 2' - 5'-oligoadénylate synthétase 1 ; le facteur-1 associé à la protéine d'apoptose (XIAP) ; le tétratricopeptide 5 induit par l'interféron ; la 2' - 5' -oligoadénylate de type synthétase ; la 2' -5'-oligoadénylate synthétase 3 ; le tétratricopeptide 1 induit par l'interféron ; la scramblase phospholipidique 1 ; le domaine hect et RLD 5 ; la protéine kinase inductible par l'interféron, le membre 10 de la superfamille des TNF ; la protéine 1 se liant à la guanylate ; la protéine 6 induite par le TNF-alpha ; le tétracopeptide 3 induit par l'interféron ; le domaine de type 9 du motif alpha stérile ; la protéine 5 modifiant l'activité de la chromatine ; le modificateur de la protéine ISG15 de type ubiquitine ; la 2' -5' - oligoadénylate synthétase 2 ; le domaine 1 de l'hélicase C induit par l'interféron ; la résistance 1 au myxovirus ; et le gène 1 d'interaction de l'épithélium et du stroma.

5. Procédé selon la revendication 4, dans lequel au moins 5 gènes sont dosés.

6. Procédé selon la revendication 5, dans lequel au moins 15 gènes sont dosés.

7. Procédé selon l'une quelconque des revendications précédentes, dans lequel le dosage est réalisé en utilisant une plaque de microréseaux comprenant une série d'oligonucléotides distincts immobilisés, dans laquelle :
a) lesdits oligonucléotides s'hybrident dans des conditions strictes avec la séquence génique du gène défini à la revendication 1 ;
b) lesdits oligonucléotides sont immobilisés à une position sur ladite plaque de microréseaux qui ne contient aucun oligonucléotide qui s'hybride avec d'autres séquences dans des conditions strictes ; et
c) ladite plaque de microréseaux comprend au plus 100 oligonucléotides distincts immobilisés au total.

8. Procédé selon la revendication 7, dans lequel ladite plaque de microréseaux comprend des oligonucléotides qui s'hybrident dans des conditions strictes avec les séquences géniques d'une ou de plusieurs protéines 27 inductibles par l'interféron alpha ; la protéine de type 44 induite par l'interféron ; la protéine 44 induite par l'interféron ; la protéine LOC129607 hypothétique ; la 2'-5' -oligoadénylate synthétase 1 ; le facteur-1 associé à la protéine d'apoptose (XIAP) ; le tétratricopeptide 5 induit par l'interféron ; le 2'-5' -oligoadénylate apparenté à la synthétase ; la 2'-5' -oligoadénylate synthétase 3 ; le tétratricopeptide 1 induite par l'interféron ; la scramblase phospholipidique 1 ; le domaine hect et RLD 5 ; la protéine kinase inductible par l'interféron ; le membre 10 de la superfamille des TNF ; la protéine 1 se liant à la guanylate ; la protéine 6 induite par le TNF-alpha ; le tétratricopeptide 3 induit par l'interféron ; le domaine de type 9 du motif alpha stérile ; la protéine 5 modifiant la chromatine ; le modificateur de la protéine ISG15 de type ubiquitine ; la 2'-5' -oligoadénylate synthétase 2 ; le domaine 1 de l'hélicase C induit par l'interféron, la résistance 1 au myxovirus ; et le gène 1 d'interaction de l'épithélium et du stroma.

9. Procédé selon la revendication 8, dans lequel ladite plaque de microréseaux comprend au moins 10 des oligonucléotides distincts immobilisés, de préférence au moins 15 des oligonucléotides distincts immobilisés, et de préférence comprend des oligonucléotides distincts immobilisés qui s'hybrident dans des conditions strictes spécifiquement à tous lesdits gènes.

10. Procédé selon l'une quelconque des revendications 6 à 9, dans lequel ladite plaque de microréseaux ne comprend pas plus de 50 oligonucléotides distincts immobilisés au total.

11. Procédé selon l'une quelconque des revendications 6 à 10, dans lequel la plaque de microréseaux ne comprend aucun oligonucléotide distinct immobilisé qui s'hybride dans des conditions strictes avec un gène autre qu'undit gène.

12. Procédé selon l'une quelconque des revendications précédentes, dans lequel, si le résultat déterminé dans l'étape de dosage indique que le gène RSAD2 est exprimé au moins 20 fois plus souvent dans ledit échantillon de test que dans un ou plusieurs des échantillons témoins, il peut alors en être conclu que ledit sujet présente un profil d'expression génique qui est caractéristique de la dermatomyosite ou de la polymyosite et qui n'est pas caractéristique de l'IBM.


### SEQUENCE LISTING

<110> The Brigham and Women's Hospital, Inc.
<120> Compositions and Methods for Diagnosing and Assessing Inflammatory Myopathies
<130> REP08655EP2
<150> EP 08780008.2
   <151> 2008-07-07
<150> PCT/US2008/008333
   <151> 2008-07-07
<150> US 60/929,775
   <151> 2007-07-12
<160> 4
<170> PatentIn version 3.4
<210> 1
   <211> 20
   <212> DNA
   <213> Homo sapiens
<400> 1
   cggctaacgg ataagcagag 20
<210> 2
   <211> 20
   <212> DNA
   <213> Homo sapiens
<400> 2
   acctacagct ggctcctgaa 20
<210> 3
   <211> 20
   <212> DNA
   <213> Homo sapiens
<400> 3
   aaaagcccac atttgaggtg 20
<210> 4
   <211> 20
   <212> DNA
   <213> Homo sapiens
<400> 4
   gaaattcctg aaaccgacca 20